# EUROPÄISCHE PATENTSCHRIFT

(11) **EP 1 216 259 B1**
(45) Veröffentlichungstag und Bekanntmachung des Hinweises auf die Patenterteilung: **21.03.2007**
(21) Anmeldenummer: 00956507.8
(22) Anmeldetag: 01.09.2000
(51) Int. Cl.: C07K 14/815, C07K 14/24, C07K 14/21, C07K 14/245, C12N 9/02, C12N 15/62, C12N 1/21, A61P 7/02

(54) **SIGNALSEQUENZEN ZUR HERSTELLUNG VON LEU-HIRUDIN ÜBER SEKRETION DURCH E. COLI IN DAS KULTURMEDIUM**
SIGNAL SEQUENCES FOR THE PRODUCTION OF LEU-HIRUDINE VIA SECRETION BY E. COLI IN A CULTURE MEDIUM
SEQUENCES DE SIGNAUX POUR LA FABRICATION DE LEU-HIRUDINE AU MOYEN DE SECRETION PAR E. COLI DANS LE MILIEU DE CULTURE

(30) Priorität: 18.09.1999 DE 19944870
(43) Veröffentlichungstag der Anmeldung: 26.06.2002
(73) Patentinhaber: Sanofi-Aventis Deutschland GmbH, 65929 Frankfurt am Main (DE)
(72) Erfinder: HABERMANN, Paul, 65817 Eppstein (DE); BENDER, Rudolf, 65812 Bad Soden (DE)
(86) Internationale Anmeldenummer: PCT/EP2000/008537
(87) Internationale Veröffentlichungsnummer: WO 2001/021662

(56) Entgegenhaltungen:
- EP-A- 0 324 712
- EP-A- 0 448 093
- EP-A- 0 511 393
- US-A- 5 389 529
- US-A- 5 652 139
- DE MOT R ET AL: "HOMOLOGY OF THE ROOT ADHESIN OF PSEUDOMONAS-FLUORESCENS OE 28.3 WITH PORIN F OF PSEUDOMONAS-AERUGINOSA AND PSEUDOMONAS-SYRINGAE" MOLECULAR & GENERAL GENETICS, Bd. 231, Nr. 3, 1992, Seiten 489-493, XP000982041 ISSN: 0026-8925
- BRAUN G ET AL: "DNA SEQUENCE ANALYSIS OF THE SERRATIA-MARCESCENS OMP-A GENE IMPLICATIONS FOR THE ORGANIZATION OF AN ENTEROBACTERIAL OUTER MEMBRANE PROTEIN" MOLECULAR & GENERAL GENETICS, Bd. 195, Nr. 1-2, 1984, Seiten 321-328, XP000986721 ISSN: 0026-8925
- PEALING SARA L ET AL: "Sequence of the gene encoding flavocytochrome c from Shewanella putrefaciens: A tetraheme flavoenzyme that is a soluble fumarate reductase related to the membrane-bound enzymes from other bacteria." BIOCHEMISTRY, Bd. 31, Nr. 48, 1992, Seiten 12132-12140, XP000982039 ISSN: 0006-2960

### SEQUENZPROTOKOLL

<110> Aventis Pharma Deutschland GmbH
<120> Signalsequenzen zur Herstellung von Leu-Hirudin über Sekretion durch E.coli in das Kulturmedium
<130> 1999/L055
<140> 19944870.1
   <141> 1999-09-18
<160> 33
<170> PatentIn Ver. 2.1
<210> 1
   <211> 31
   <212> DNA
   <213> Künstliche Sequenz
<220>
   <223> Beschreibung der künstlichen Sequenz: misc feature
<400> 1
   tttttttaag cttgggctgc aggtcsdnhn d 31
<210> 2
   <211> 54
   <212> DNA
   <213> Künstliche Sequenz
<220>
   <223> Beschreibung der künstlichen Sequenz: misc feature
<400> 2
   tggcactggc aggtttcgct accgtagcgc aagcccttac gtatactgac tgca 54
<210> 3
   <211> 57
   <212> DNA
   <213> Künstliche Sequenz
<220>
   <223> Beschreibung der künstlichen Sequenz: misc feature
<400> 3
   ttttttgaat tcatgaaaaa gacagctatc gcattagcag tggcactggc aggtttc 57
<210> 4
   <211> 58
   <212> DNA
   <213> Künstliche Sequenz
<220>
   <223> Beschreibung der künstlichen Sequenz: misc feature
<400> 4
   ggttctctta ttgccgctac ttctttcggc gttctggcac ttacgtatac tgactgca 58
<210> 5
   <211> 56
   <212> DNA
   <213> Künstliche Sequenz
<220>
   <223> Beschreibung der künstlichen Sequenz: misc feature
<400> 5
   ttttttgaat tcatgaaaaa caccttgggc ttggccattg gttctcttat tgccgc 56
<210> 6
   <211> 61
   <212> DNA
   <213> Künstliche Sequenz
<220>
   <223> Beschreibung der künstlichen Sequenz: misc feature
<400> 6
   gttgccgtcg cagcgggcgt aatgtctgct caggcaatgg ctcttacgta tactgactgc 60
   a 61
<210> 7
   <211> 59
   <212> DNA
   <213> Künstliche Sequenz
<220>
   <223> Beschreibung der künstlichen Sequenz: misc feature
<400> 7
   ttttttgaat tcatgatgat tactctgcgc aaacttcctc tggcggttgc cgtcgcagc 59
<210> 8
   <211> 63
   <212> DNA
   <213> Künstliche Sequenz
<220>
   <223> Beschreibung der künstlichen Sequenz: misc feature
<400> 8
   ctaccctgat gggtaccgct ggtctgatgg gtaccgctgt tgctcttacg tatactgact 60
   gca 63
<210> 9
   <211> 60
   <212> DNA
   <213> Künstliche Sequenz
<220>
   <223> Beschreibung der künstlichen Sequenz: misc feature
<400> 9
   ttttttgaat tcatgaaaaa aatgaacctg gctgtttgca tcgctaccct gatgggtacc 60
<210> 10
   <211> 61
   <212> DNA
   <213> Künstliche Sequenz
<220>
   <223> Beschreibung der künstlichen Sequenz: misc feature
<400> 10
   ctgatcccgt tctttgcagc gttctgcctg ccggttttcg cgcttacgta tactgactgc 60
   a 61
<210> 11
   <211> 56
   <212> DNA
   <213> Künstliche Sequenz
<220>
   <223> Beschreibung der künstlichen Sequenz: misc feature
<400> 11
   ttttttgaat tcatgtccat ccagcacttc cgcgtcgccc tgatcccgtt ctttgc 56
<210> 12
   <211> 53
   <212> DNA
   <213> Künstliche Sequenz
<220>
   <223> Beschreibung der künstlichen Sequenz: misc feature
<400> 12
   gctgccgctg ctgttcaccc cggttaccaa agcgcttacg tatactgact gca 53
<210> 13
   <211> 57
   <212> DNA
   <213> Künstliche Sequenz
<220>
   <223> Beschreibung der künstlichen Sequenz: misc feature
<400> 13
   ttttttgaat tcatgaaaca gtcgaccatc gcgctggcgc tgctgccgct gctgttc 57
<210> 14
   <211> 53
   <212> DNA
   <213> Künstliche Sequenz
<220>
   <223> Beschreibung der künstlichen Sequenz: misc feature
<400> 14
   gctgagctgc ctgatcaccc cggtgtccca ggcgcttacg tatactgact gca 53
<210> 15
   <211> 57
   <212> DNA
   <213> Künstliche Sequenz
<220>
   <223> Beschreibung der künstlichen Sequenz: misc feature
<400> 15
   ttttttgaat tcatgaaaca gagcgcgatc gcgctggctc tgctgagctg cctgatc 57
<210> 16
   <211> 64
   <212> DNA
   <213> Künstliche Sequenz
<220>
   <223> Beschreibung der künstlichen Sequenz: misc feature
<400> 16
   ctttcgctga gtatggcgtt ggggatttca ctgcccgcat gggcacttac gtatactgac 60
   tgca 64
<210> 17
   <211> 65
   <212> DNA
   <213> Künstliche Sequenz
<220>
   <223> Beschreibung der künstlichen Sequenz: misc feature
<400> 17
   ttttttgaat tcatgaaatc gcggtacaaa cgtttgacct ccctggcgct ttcgctgagt 60
   atggc 65
<210> 18
   <211> 55
   <212> DNA
   <213> Künstliche Sequenz
<220>
   <223> Beschreibung der künstlichen Sequenz: misc feature
<400> 18
   tggtttcagc tttagtaagc ggggttgcat ttgctcttac gtatactgac tgcac 55
<210> 19
   <211> 60
   <212> DNA
<213> Künstliche Sequenz
<220>
   <223> Beschreibung der künstlichen Sequenz: misc feature
<400> 19
   ttttgggaat tcatgaaaaa gacaattatg tctctggctg tggtttcagc tttagtaagc 60
<210> 20
   <211> 60
   <212> DNA
   <213> Künstliche Sequenz
<220>
   <223> Beschreibung der künstlichen Sequenz: misc feature
<400> 20
   cggcgctgag tctcgcctta ttttctcacc tatcttttgc ccttacgtat actgactgca 60
<210> 21
   <211> 57
   <212> DNA
   <213> Künstliche Sequenz
<220>
   <223> Beschreibung der künstlichen Sequenz: misc feature
<400> 21
   ttttttgaat tcatgtcatt tcatcaccgg gtatttaaac tgtcggcgct gagtctc 57
<210> 22
   <211> 267
   <212> DNA
   <213> Künstliche Sequenz
<220>
   <223> Beschreibung der künstlichen Sequenz: gene
<400> 22
   cttacgtata ctgactgcac tgaatctggt cagaacctgt gcctgtgcga aggatctaac 60
   tytdctsgnc cgsngtttgc ggccagggta acaaatgcat ccttggatcc gacggtgaaa 120
   agaaccagtg cgttvcggnk cgsdgkncva ctggcgaagg taccccgaaa ccgcagtctc 180
   ataacgacgg cgacttcgaa gagatccctt ggtkshndgd gaggaatacc ttcagtaata 240
   gagctcgtcg acctgcagcc caagctt                                     267
<210> 23
   <211> 5
   <212> PRT
   <213> Künstliche Sequenz
<220>
   <223> Beschreibung der künstlichen Sequenz: peptide
<400> 23
   Glu Glu Tyr Leu Gln
   1               5
<210> 24
   <211> 30
   <212> PRT
   <213> Künstliche Sequenz
<220>
   <223> Beschreibung der künstlichen Sequenz: signal
<400> 24
   Met Lys Arg Asn Arg Phe Phe Asn Thr Ser Ala Ala Ile Ala Ile Ser
   1               5                   10                  15
   Ile Ala Leu Asn Thr Phe Phe Cys Ser Met Gln Thr Ile Ala
               20                  25                  30
<210> 25
   <211> 21
   <212> PRT
   <213> Künstliche Sequenz
<220>
   <223> Beschreibung der künstlichen Sequenz: signal
<400> 25
   Met Lys Lys Thr Ala Ile Ala Leu Ala Val Ala Leu Ala Gly Phe Ala
   1               5                   10                  15
   Thr Val Ala Gln Ala
               20
<210> 26
   <211> 22
   <212> PRT
   <213> Künstliche Sequenz
<220>
   <223> Beschreibung der künstlichen Sequenz: signal
<400> 26
   Met Lys Asn Thr Leu Gly Leu Ala Ile Gly Ser Leu Ile Ala Ala Thr
    1               5                  10                  15
   Ser Phe Gly Val Leu Ala
                20
<210> 27
   <211> 25
   <212> PRT
   <213> Künstliche Sequenz
<220>
   <223> Beschreibung der künstlichen Sequenz: signal
<400> 27
   Met Met Ile Thr Leu Arg Lys Leu Pro Leu Ala Val Ala Val Ala Ala
    1               5                  10                  15
   Gly Val Met Ser Ala Gln Ala Met Ala
                20                  25
<210> 28
   <211> 25
   <212> PRT
   <213> Künstliche Sequenz
<220>
   <223> Beschreibung der künstlichen Sequenz: signal
<400> 28
   Met Lys Lys Met Asn Leu Ala Val Cys Ile Ala Thr Leu Met Gly Thr
    1               5                  10                  15
   Ala Gly Leu Met Gly Thr Ala Val Ala
                      20                  25
<210> 29
   <211> 23
   <212> PRT
   <213> Künstliche Sequenz
<220>
   <223> Beschreibung der künstlichen Sequenz: signal
<400> 29
   Met Ser Ile Gln His Phe Arg Val Ala Leu Ile Pro Phe Phe Ala Ala
    1               5                  10                  15
   Phe Ser Leu Pro Val Phe Ala
               20
<210> 30
   <211> 21
   <212> PRT
   <213> Künstliche Sequenz
<220>
   <223> Beschreibung der künstlichen Sequenz: signal
<400> 30
   Met Lys Gln Ser Thr Ile Ala Leu Ala Leu Leu Pro Leu Leu Phe Thr
    1               5                  10                  15
   Pro Val Thr Lys Ala
               20
<210> 31
   <211> 21
   <212> PRT
   <213> Künstliche Sequenz
<220>
   <223> Beschreibung der künstlichen Sequenz: signal
<400> 31
   Met Lys Gln Ser Ala Ile Ala Leu Ala Leu Leu Ser Cys Leu Ile Thr
    1               5                  10                  15
   Pro Val Ser Gln Ala
               20
<210> 32
   <211> 27
   <212> PRT
   <213> Künstliche Sequenz
<220>
   <223> Beschreibung der künstlichen Sequenz: signal
<400> 32
   Met Lys Ser Arg Tyr Lys Arg Leu Thr Ser Leu Ala Leu Ser Leu Ser
    1               5                  10                  15
   Met Ala Leu Gly Ile Ser Leu Pro Ala Trp Ala
               20                  25
<210> 33
   <211> 24
   <212> PRT
   <213> Künstliche Sequenz
<220>
   <223> Beschreibung der künstlichen Sequenz: signal
<400> 33
   Met Ser Phe His His Arg Val Phe Lys Leu Ser Ala Leu Ser Leu Ala
    1               5                  10                  15
   Leu Phe Ser His Leu Ser Phe Ala
               20

## Beschreibung

Das vom Blutegel abgeleitete Präparat Refludan^{®} zeigt in der klinischen Prüfung gute therapeutische Eigenschaften (The Lancet, Vol.353, p.429 - 438). Dies läßt den Schluß zu, daß in Zukunft ein größerer Mengenbedarf für das Präparat zu erwarten ist. Der biologische aktive Wirkstoff des Präparates ist das in dem europäischen Patent 0 324 712 beschriebene [Leu¹, Thr²]-63-Desulfato-hirudin, im folgenden kurz "Leu-Hirudin" genannt.

In dem europäischen Patent 0 448 093 ist ein Verfahren zur Herstellung von Hirudin beschrieben. Die bevorzugte Ausführung des Patentes umfaßt ein Hirudin, dessen N-terminale Aminosäure aus Alanin besteht. Fusioniert man dieses Hirudin mit der Signalsequenz der α-Cyclodextringlykosyltransferase (CGTase) und transformiert einen dieses Fusionsprotein kodierenden Expressionsvektor , wie in dem Patent beschrieben, in eine *E*. *coli* Sekretormutante, so kann Ala -Hirudin mit Rohausbeuten von größer 2 Gramm pro Liter hergestellt werden. Das europäische Patent 0 549 915 beschreibt Varianten des Ala - Hirudin mit verbesserter Stabilität. Werden diese Varianten mit dem *E*. *coli* Sekretor System hergestellt, so ergeben sich Ausbeuten von mehreren Gramm pro Liter. Die Ausbeuten sind damit deutlich höher als dies von Dodt *et.al.* für die Hirudinvariante HV1 beschrieben wurde (FEBS LETTERS vol. 202 373 -377, 1986). Eine im Vergleich dazu unwesentliche Steigerung der Ausbeute wird im US Patent 5,573,929 beschrieben, indem anstelle des von Dodt *et al.* pBR322 abgeleiteten Vektors in bekannter Weise die Expressionskassette über einen pUC - Vektor exprimiert wird. Bender *et al.* (Appl. Microbiol Biotechnol 34, p.203 -207 1990) beschreiben die Sekretion im europäischen Patent 0 171 024 beschriebenen Thr - Hirudins durch *Streptomyces lividans.* Aber auch hier sind die Ausbeuten im Vergleich zu denen in den europäischen Patenten 0 448 093 und 0 549 915 genannten Ausbeuten deutlich geringer. Dies gilt auch für die Expression in *E.coli* B, wie sie P. de Taxis du Poet *et al*. für die Sekretion der Hirudinvariante HV1 über die Signalsequenz Ompa von *E*. *coli* beobachten. Die Autoren finden Ausbeuten von 300mg/l Hirudin im Periplasma und ca. 40 mg/ I im Zellüberstand. Die in dem Artikel gleichzeitig beschriebene Expression in Insektenzellsystemen ist gering (400 µg/l).

Mit den Hefeexpressionsystemen *Hansenula polymorpha* oder *Pichia pastoris* erzielbaren Ausbeuten kommen den in den europäischen Patenten 0 448 093 und 0 549 915 beschriebenen Ausbeuten im Gegensatz zu denen mit *S*. *cerevisiae* erzielten Werten am nächsten.

Rosenfeld *et al.* (Protein Expression and Purification 8 , 476 - 482, 1996) beschreiben die Expression und Sekretion von Hirudin durch die Hefe *Pichia pastoris*. Dabei werden Ausbeuten von ca. 1,5g/l Kulturbrühe erreicht. Eine ähnliche Größenordnung läßt sich mit der Hefe *Hansenula polymorpha* erreichen (Appl. Microbiol. Biotechnol. 44, 377 - 385 1995). Ein erheblicher Nachteil solcher Expressionssysteme liegt aber in deutlich längeren Fermentationszeiten gegenüber dem *E.coli* System. Es wäre also vorteilhaft, wenn Leu -Hirudin wie Ala -Hirudin über Sekretion durch *E.coli* herstellbar wäre.

Dies gelingt aber nicht mit dem in dem europäischen Patent 0 448 093 beschriebenen System. Deswegen wird in dem Patent vorgeschlagen, die Leu - Hirudin- Sequenz um das Tripeptid Ala - Thr - Arg zu verlängern, so daß ein Prä - Leu - Hirudin entsteht, das schließlich nach Umsetzung mit Trypsin zu dem nativen Wirkstoff Leu - Hirudin umgewandelt wird. Folgt man diesem Vorschlag , so ergeben sich bereits deutlich schlechtere Rohausbeuten im Schüttelkolbenexperiment, als für Ala-Hirudin beschrieben wurde. Damit ist ein eindeutiger Vorteil gegenüber späteren Hefeexpressionssystemen zunächst nicht mehr klar ersichtlich.

Die der vorliegenden Erfindung zugrundeliegende Aufgabe war es demgemäß, ein Fusionsprotein herzustellen, bei dem die Kombination aus Signalsequenz und Leu - Hirudin, die direkte Prozessierung zu Leu-Hirudin und anschließende Sekretion von nativem Leu-Hirudin in hohen Ausbeuten durch *E. coli* erlaubt. Dies ist die Voraussetzung zur Entwicklung eines Verfahrens, das sich sowohl in der Fermentation als auch in der sich anschließenden Reinigung durch die verbesserte Ausgangskonzentration des Hirudin vorteilhaft auf die Herstellkosten von Refludan auswirkt.
Es wurde nun überraschend gefunden, daß Signalsequenzen existieren , die eine direkte Sekretion von Leu-Hirudin durch *E. coli* erlauben und daß dabei sogar eine effizientere Sekretion als im europäischen Patent 0 448 093 beschrieben wurde, beobachtet wird. Damit kann ein Verfahren entwickelt werden, das ohne großen Aufwand hohe Mengen an Leu-Hirudin zugänglich werden läßt. Dies ist Gegenstand der Erfindung.

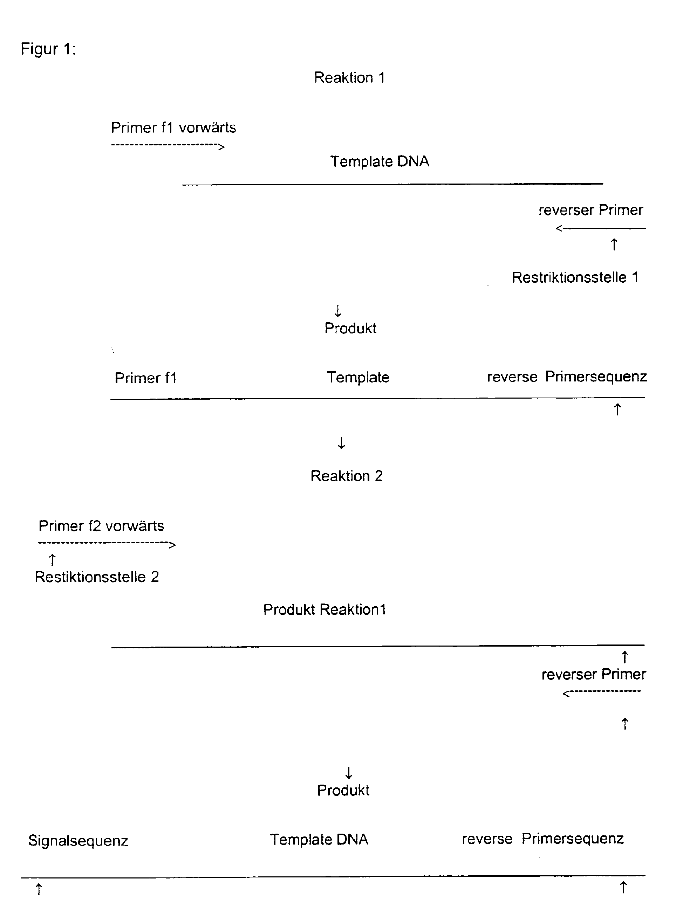

Um vorteilhafte Signalsequenzen zu finden, wird eine Methode des PCR gestützten Signalsequenz - Screenings eingeführt. Diese Methode benutzt die das Protein von Interesse kodierende DNA als Matrize und einen definierten reversen PCR -Primer, sowie variable vorwärts gerichtete Primer, die die Synthese eines DNA-Abschnittes, der eine Signalsequenz gekoppelt an ein Gen von Interesse kodiert, erlauben. Die Reaktion läuft nach dem in Figur 1 dargestellten Schema ab. Es ist dem Fachmann klar, daß entsprechend der Länge der zu synthetisierenden Signalsequenz die Zahl der Reaktionsschritte variieren kann. Kurze Signalsequenzen können mit einem Reaktionsschritt, längere Sequenzen mit zwei, drei oder mehr Reaktionen hergestellt werden. Zudem ist die Zahl der Reaktionen auch abhängig von dem zur Synthese der als Primer benutzten Oligonukleotide verwendeten Gerät. Die so synthetisierte Signalpeptid - Genfusion kann dann gezielt mit den die Restriktionsstellen 1 und 2 erkennenden Enzymen gespalten werden und in einen entsprechend geöffneten Expressionsvektor insertiert werden. Von allgemeiner Bedeutung wird das System dann, wenn man als Gen von Interesse Hirudin wählt. Die N - terminale Aminosäure von Hirudin kann dabei variabel gewählt werden. Dies führt zwar zu einer gewissen Beeinflussung der Bindung von Hirudin an Thrombin (Veränderung der Bindungskonstante), jedoch bleibt der inhibitorische Effekt von Hirudin bezüglich der Thrombinaktivität meßbar.

Die Patentschrift EP-B1 0 448 093 beschreibt die Sekretion von Hirudin in den Kulturüberstand. Dort ist die Hirudinkonzentration über den bekannten Thrombinhemmtest direkt bestimmbar. Die Hirudinkonzentration ist ein direktes Maß für die Effizienz der Sekretion und damit der Abspaltung der Signalsequenz. Das Patent beschreibt aber, daß z.B. Hirudin beginnend mit der Aminosäure Leucin nicht effizient über die Signalsequenz der CGT-ase in den Überstand abgegeben werden kann. Mit Hilfe der oben beschriebenen Methode kann man nun nach Signalsequenzen suchen, die dies effektiv erlauben. In ähnlicher Weise kann man nun die Sekretion von Hirudinen, die mit einer der übrigen 19 Aminosäuren beginnen, untersuchen. Man erhält so jeweils ein Spektrum von Signalsequenzen, die modellhaft die effiziente Prozessierung der carboxyterminalen Aminosäure des Signalpeptides und daran anknüpfenden peptidischen Restes erlauben. Damit kann man eine Vorauswahl an Signalpeptiden zur effizienten Sekretion eines beliebigen Proteines in das Periplasma treffen und so die Chance zur Entwicklung eines vorteilhaften Herstellprozesses für ein Protein erhöhen. Dies ist ebenfalls Gegenstand der Erfindung. Man kann das Verfahren beschleunigen bzw. automatisieren, indem man das Transformationsgemisch aus Ligationsansatz und kompetenten Zellen als Flüssigkultur in einem Selektionsmedium über Nacht schüttelt und am nächsten Tag mit einem Aliquot der Zellen wie in Beispiel 11 beschrieben Medium, das Induktor enthält zur Durchführung der Induktion beimpft aber den größten Teil der Kultur zentrifugiert und das Zellpellet wegfriert. Findet man bei der Expression Hirudinaktivität, so kann man das entsprechende Expressionsplasmid aus den Zellen reisolieren, linearisieren und gelelektrophoretisch von etwaigen Autoligationsprodukten separieren. Die lineare Plasmid DNA wird dann religiert und erneut in den Wirtsstamm transformiert. Nun kann man einzelne Kolonien isolieren und auf ihre Expressionsleistung testen. Dabei kann man so vorgehen, daß das Verfahren Kriterien der Arzneimittelzulassung erfüllt.

Ein weiterer Vorteil des Vorgehens besteht darin, daß man verschiedenen Varianten eines Signalpeptides, wie sie im Lauf der Evolution durch Austausch von Aminosäuren zwischen einzelnen Spezies entstanden sind, leicht nebeneinander im Hinblick auf ihre Tauglichkeit zur effizienten Sekretion eines Hirudins untersuchen kann.

Auch ist das Verfahren vorteilhaft gegenüber des Einsatzes von Computer Programmen wie von Nielsen *et al*. (Protein Engineering 10, 1-6, 1997) beschrieben, mit deren Hilfe sich Schnittstellen zwischen Signalsequenz und einem Protein von Interesse vorhersagen lassen. Es zeigt sich jedoch, daß die hiermit zu treffenden Voraussagen nicht in jedem Fall zutreffen, so daß leicht vorteilhafte Kombinationen übersehen werden könnten. Zudem besteht zwischen der Voraussage der korrekten Prozessierung und der tatsächlich erzielbaren Ausbeute keine Beziehung.

Ein Gegenstand der Erfindung ist ein Hirudinvorläufer enthaltend eine Signalsequenz ausgewählt aus der Gruppe enthaltend die Signalsequenzen des äußeren Membranproteins von *Serratia marcescens,* des oprF-Proteins von *Pseudomonas fluorescens* und der Fumarat-Reduktase von *Shewanella putrifaciens,* vorzugsweise ausgewählt wird aus der Gruppe enthaltend die Signalsequenz des äußeren Membranproteins von *Serratia marcescens* und der Fumarat Reduktase von *Shewanella putrifaciens.* an welche C - terminal die Sequenz von Leu-Hirudin angefügt ist.

Ein weiterer Gegenstand der Erfindung ist ein Verfahren zur Herstellung von Leu-Hirudin, bei welchem als Zwischenstufe ein Hirudinvorläufer wie oben beschrieben vorkommt, bei dem
(a) ein Expressionsplasmid enthaltend eine DNA-Sequenz kodierend für den Hirudinvorläufer hergestellt wird;
(b) das Expressionsplasmid gemäß (a) in einer geeigneten *E*. *coli* Zelle exprimiert wird;
(c) der Hirudinvorläufer aus *E. coli* sekretiert und gleichzeitig prozessiert wird; und
(d) das Leu-Hirudin direkt aus dem Kulturmedium isoliert wird.

Ebenfalls Gegenstand der Erfindung ist die Verwendung eines Hirudinvorläufers wie oben beschrieben zur Herstellung von Leu-Hirudin, vorzugsweise in einem Verfahren wie oben beschrieben.

Beispielhaft soll die Synthese von Signalsequenzen, die die effiziente Synthese und Sekretion von Leu-Hirudin erlauben, beschrieben werden. Ebenfalls wird die Synthese anderer Signalsequenzen, die nicht oder hinsichtlich der Ausbeute mit schlechteren Ergebnissen zum Ziel führten, beschrieben. Die Beispiele sollen dabei den Gedanken der Erfindung anhand der Auswahl von Signalsequenzen anhand von Leu-Hirudin erläutern, jedoch nicht darauf beschränkt sein.

Die beschriebenen Verfahren können zur Aufreinigung von Refludan verwendet werden; dies ist beispielsweise in Beispiel 11 beschrieben.

### Beispiel 1 : Synthese eines Fusionsgenes kodierend für ein Fusionsprotein bestehend aus Leu-Hirudin und der Signalsequenz des äußeren Membranproteins aus Serratia marcescens.

Als Expressionsplasmid wird der im europäischen Patent 0 468 539 in Figur 1 beschriebene Vektor pJF118 verwendet, da dieser bzgl. seines Grundgerüstes mit dem im europäischen Patent 0 448 093 beschriebenen Vektor pCM7053 identisch ist.

Als Matrize wird das im europäischen Patent 0 448 093 in Beispiel 1 genannte Plasmid pK152 verwendet, das die Hirudinsequenz entsprechend dem europäischen Patent 0 171 024 trägt.

Das Membranprotein wurde von Braun, G. und Cole, S.T: (Mol.Gen.Genet. 195, 321-328 , 1984) beschrieben.

Zur Synthese des gewünschten DNA - Abschnittes werden drei Oügonukleotidsequenzen hergestellt.

Oligonukleotid hirrev hat die Sequenz :

Der Primer hybridisiert gegen die Region 227 -210 bp des in der Tabelle 1 dargestellten Hirudingenes.

Primer smompaf1 hat die Sequenz:
5'-TGGCACTGGC AGGTTTCGCT ACCGTAGCGC AAGCCcttac gtatactgac tgca - 3' [SEQ ID NO: 2]

Der Primer hybridisiert gegen die Nukleotide 1-19 der in Tabelle 1 dargestellten Hirudinsequenz. Der hybridisierende Teil der Primersequenz ist mit kleinen Buchstaben symbolisiert. Der Rest der Sequenz hybridisiert gegen die Region 229bp -263 bp der von Braun,G. und Cole,S.T. (Mol. Gen. Genet. 195, 321-328, 1984) publizierten Sequenz.

Primer smompaf2 hat die Sequenz :
5'- ttttttgaat tcATGAAAAA GACAGCTATC GCATTAGCAG TGGCACTGGC AGGTTTC - 3' [SEQ ID NO: 3]

Ab Position 13 bp hybridisiert die Primersequenz mit der von Braun und Cole publizierten Sequenz von 201 bp - 245bp und überlappt somit mit der Primersequenz smompaf2. Die Position 1- 12 des Primers enthält eine Erkennungsstelle für das Restriktionsenzym *Eco*RI sowie angrenzend sechs T-Nukleotide, um die Erkennung durch das Enzym zu ermöglichen.

In einer Standard - PCR (wie z.B. 94°C :10",50 °C: 30",72°C: 45", 25 Zyklen) mit DNA des Plasmides pK152 als Matrize, das die in Tabelle 1 beschriebene Sequenz trägt, und den Primern hirrev und smompaf1 wird die Hirudinsequenz um die bakterielle Teilsignalsequenz verlängert. Das Reaktionsprodukt wird dann in einer zweiten PCR als Template mit den Primern hirrev und smompaf2 unter gleichen Bedingungen umgesetzt. Als Reaktionsprodukt entsteht ein DNA-Fragment, das für ein Fusionsprotein kodiert, welches aus der um die gewünschte Signalsequenz verlängerten Hirudinsequenz besteht. Am 5'Ende findet sich die Erkennungsstelle für das Restriktionsenzym *Eco*RI und am 3' Ende die Erkennungsstelle für das Enzym *Hin*dIII.

Das Reaktionsprodukt der zweiten PCR wird in einem Doppelverdauansatz mit den beiden Restriktionsenzyme umgesetzt und als *Eco*RI/ *Hin*dIII Fragment in die mit den beiden Enzymen geöffnete Vektor DNA in einer T4 - DNA - Ligasereaktion insertiert. Kompetente Zellen des Stammes *E*. *coli* Mc1061 oder der Sekretor - Mutante WCM100 werden mit dem Ligationsgemisch transformiert und unter Selektionsdruck auf Ampicillin-haltigen Platten vermehrt. Am nächsten Morgen erfolgt dann die Expression gemäß Beispiel 6 im Vergleich zur Ala-Hirudinexpression mit dem Stamm *E*. *coli* WCM 100 / pCM7053. Es zeigt sich , daß die erzielte Expression ca. 1,5 mal besser ist als im Vergleichsversuch.

### Beispiel 2 : Synthese des Fusionsproteins aus Leu-Hirudin und der Signalsequenz des oprF - Genproduktes aus Pseudomonas fluorescens.

Die Konstruktion erfolgt entsprechend dem in Beispiel 1 beschriebenen Schema mit Ausnahme, daß anstelle der Primer smompaf1 / f2 zwei neue Primer benutzt werden, die bezüglich ihrer Spezifität für das Hirudingen und der Sequenz zur Erkennung durch das Restriktionsenzym *Eco*RI die gleichen Merkmale wie die smompa -Primer aufweisen, aber für die gewünschte Signalsequenz des oprF - Genes (De, E. *et al.:* FEMS Microbial. Lett. 127, 267 -272 , 1995) kodieren.

Primer pfuf1 hat die Sequenz:
5'GGTTCTCTTA TTGCCGCTAC TTCTTTCGGC GTTCTGGCAc ttacgtatac tgactgca 3'0 [SEQ ID NO: 4]

Primer pfuf2 hat die Sequenz :
5'ttttttgaat tcatgAAAAA CACCTTGGGC TTGGCCATTG GTTCTCTTAT TGCCGC 3' [SEQ ID NO: 5]

Dabei wird der Primer pfuf1 entsprechend Beispiel 1 in PCR1 und der Primer pfuf2 entsprechend in der PCR2 verwendet. Die Expression wird im Vergleich zur Ala- Hirudinexpression mit dem Stamm *E. coli* WCM100 / pCM7053 durchgeführt. Es zeigt sich, daß die erzielte Expression ca. 1,1 mal besser ist , als im Vergleichsversuch. Nach gelelektrophoretischer Auftrennung im SDS- PAGE System wird die Hirudinbande isoliert und die N- terminale Sequenz des Hirudins bestimmt. Es zeigt sich , daß die Sequenz vollständig intakt ist und mit der Aminosäure Leucin beginnt. Dieses Ergebnis ist überraschend, da das Programm zur Identifikation der putativen Signalpeptidaseerkennungsstelle eine Verlängerung des Hirudins um Valin voraussagt.

### Beispiel 3 : Synthese des Fusionsproteins aus Leu-Hirudin und der Signalsequenz des lamB Genproduktes aus E. coli

Die Konstruktion erfolgt entsprechend dem in Beispiel 1 beschriebenen Schema mit Ausnahme, daß anstelle der Primer smompaf1 / f2 zwei neue Primer benutzt werden, die bezüglich ihrer Spezifität für das Hirudingen und der Sequenz zur Erkennung durch das Restriktionsenzym *Eco*RI die gleichen Merkmale wie die smompa -Primer aufweisen, aber für die gewünschte Signalsequenz des lamb - Genes (Clement,J.M. and Hofnung, M. : Cell 27, 507 -514, 1981) kodieren.

Primer lambbf1 hat die Sequenz:
5' GTTGCCGTCG CAGCGGGCGT AATGTCTGCT CAGGCAATGG CTcttacgta tactgactgc a 3' [SEQ ID NO: 6]

Primer lambbf2 hat die Sequenz :
5'ttttttgaat tcATGATGAT TACTCTGCGC AAACTTCCTC TGGCGGTTGC CGTCGCAGC 3' [SEQ ID NO: 7]

Dabei wird der Primer lambbf11 entsprechend Beispiel 1 in PCR1 und der Primer lambbf2 entsprechend in der PCR2 verwendet. Die Expression wird im Vergleich zur Ala- Hirudinexpression mit Stamm *E*. *coli* WCM100 / pCM7053 durchgeführt. Es zeigt sich , daß die erzielte Expression gleich hoch ist wie im Vergleichsversuch. Nach gelelektrophoretischer Auftrennung im SDS- PAGE System wird die Hirudinbande isoliert und die N- terminale Sequenz des Hirudins bestimmt. Es zeigt sich , daß die Sequenz vollständig intakt ist und mit der Aminosäure Leucin beginnt. Dieses Ergebnis ist überraschend, da das Programm zur Identifikation der putativen Signalpeptidaseerkennungsstelle die korrekte Prozessierung von Hirudin nicht voraussagt.

### Beispiel 4: Synthese des Fusionsproteins aus Leu-Hirudin und der Signalsequenz des Vorläufers der Fumarat Reduktase Flavoprotein Untereinheit aus Shewanella putrefaciens

Die Konstruktion erfolgt entsprechend dem in Beispiel 1 beschriebenen Schema mit Ausnahme, daß anstelle der Primer smompaf1 / f2 zwei neue Primer benutzt werden, die bezüglich ihrer Spezifität für das Hirudingen und der Sequenz zur Erkennung durch das Restriktionsenzym *Eco*RI die gleichen Merkmale wie die smompa -Primer aufweisen, aber für die gewünschte Signalsequenz aus *Shewanella putrefaciens* (Pealing S.L. et al.: Biochemistry 31, 12132 - 12140, 1992) kodieren. Da die Publikation nur die Proteinsequenz beschreibt, wird die Aminosäuresequenz entsprechend der Codon - Tabellen in eine DNA - Sequenz übersetzt, so daß sich für den

Primer spfccf1 folgende Sequenz ergibt:
5' CTACCCTGAT GGGTACCGCT GGTCTGATGG GTACCGCTGT TGCTcttacg tatactgact gca 3' [SEQ ID NO: 8]

Primer spfccf2 hat die Sequenz :
5' ttttttgaat tcATGAAAAA AATGAACCTG GCTGTTTGCA TCGCTACCCT GATGGGTACC 3' [SEQ ID NO: 9]

Dabei wird der Primer spfccf1 entsprechend Beispiel 1 in der PCR1 und der Primer spfccf2 entsprechend in der PCR2 verwendet.Die Expression wird im Vergleich zur Ala- Hirudinexpression mit Stamm *E. coli* WCM100 / pCM7053 durchgeführt. Es zeigt sich, daß die erzielte Expression ca. 1,5 mal besser ist als im Vergleichsversuch. Nach gelelektrophoretischer Auftrennung im SDS PAGE System wird die Hirudinbande isoliert und die N - terminale Sequenz des Hirudins bestimmt. Es zeigt sich, daß die Sequenz vollständig intakt ist und mit der Aminosäure Leucin beginnt. Dieses Ergebnis ist überraschend, da das Programm zur Identifikation der putativen Signalpeptidaseerkennungsstelle eine Prozessierung carboxyständig zu Cystein in Position 6 der Hirudinsequenz voraussagt.

### Beispiel 5 : Synthese des Fusionsproteins aus Leu-Hirudin und der Signalsequenz des Vorläufers der β-Laktamase aus pBR322

Die Konstruktion erfolgt entsprechend dem in Beispiel 1 beschriebenen Schema mit Ausnahme, daß anstelle der Primer smompaf1 / f2 zwei neue Primer benutzt werden, die bezüglich ihrer Spezifität für das Hirudingen und der Sequenz zur Erkennung durch das Restriktionsenzym *Eco*RI die gleichen Merkmale wie die smompa -Primer aufweisen, aber für die gewünschte Signalsequenz des β-Laktamase - Vorläuferproteines (Sutcliffe J.G.;Cold Spring Harbor Symp. Quant. Biol. 43:77-90 (1978)) kodieren.

Primer blatf1 hat folgende Sequenz :
5' CTGATCCCGT TCTTTGCAGC GTTCTGCCTG CCGGTTTTCG CGcttacgta tactgactgc a 3' [SEQ ID NO: 10]

Primer blatf2 hat die Sequenz :
5' ttttttgaat tcATGTCCAT CCAGCACTTC CGCGTCGCCC TGATCCCGTT CTTTGC 3' [SEQ ID NO: 11]

Dabei wird der Primer blatf1 entsprechend Beispiel 1 in der PCR1 und der Primer blatf2 entsprechend in der PCR2 verwendet. Die Expression wird im Vergleich zur Ala-Hirudinexpression mit Stamm *E*. *coli* WCM100 / pCM7053 durchgeführt. Es zeigt sich , daß die erzielte Expressionausbeute nur 50% - 90% der im Vergleichsversuch erzielten Ausbeute beträgt. Nach gelektrophoretischer Auftrennung im SDS PAGE System wird die Hirudinbande isoliert und die N- terminale Sequenz des Hirudins bestimmt. Es zeigt sich , daß die Sequenz vollständig intakt ist und mit der Aminosäure Leucin beginnt. Dieses Ergebnis wurde durch das Programm zur Identifikation der putativen Signalpeptiderkennungsstelle vorausgesagt.

### Beispiel 6 : Synthese des Fusionsgenes aus Leu-Hirudin und der Signalsequenz des Vorläufers der alkalischen Phosphatase aus E. coli

Die Konstruktion erfolgt entsprechend dem in Beispiel 1 beschriebenen Schema mit Ausnahme, daß anstelle der Primer smompaf1 / f2 zwei neue Primer benutzt werden , die bezüglich ihrer Spezifität für das Hirudingen und ihrer Sequenz zur Erkennung durch das Restriktionsenzym *Eco*RI die gleichen Merkmale wie die smompa -Primer aufweisen, aber für die gewünschte Signalsequenz des alkalischen Phosphataseproteins aus *E. coli* (Shuttleworth,H., Taylor, J. and Minton, N. Nucleic Acids Res. 14 (21), 8689 (1986)) kodieren.

Primer linkphoaf1 hat folgende Sequenz :
5' GCTGCCGCTG CTGTTCACCC CGGTTACCAA AGCGcttacg tatactgact gca 3' [SEQ ID NO.: 12]

Primer linkphoaf2 hat die Sequenz :
5' ttttttgAAT TCATGAAACA GTCGACCATC GCGCTGGCGC TGCTGCCGCT GCTGTTC 3' [SEQ ID NO.: 13]

Die beiden Primer sind bzgl. der Codonwahl für *E. coli* optimiert, d.h. sie entsprechen nicht vollständig der natürlichen Sequenz des Ausgangsgenes.

Dabei wird der Primer linkphoaf1 entsprechend Beispiel 1 in PCR1 und der Primer linkphof2 entsprechend in der PCR2 verwendet. Die Expression wird im Vergleich zur Ala - Hirudinexpression mit dem Stamm *E*. *coli* WCM100 /pCM7053 durchgeführt. Es zeigt sich, daß die erzielte Expressionausbeute nur ein Bruchteil der im Vergleichsversuch erzielten Ausbeute beträgt. Durch gelektrophoretische Auftrennung im SDS PAGE System wird die Hirudinbande isoliert und die N- terminale Sequenz des Hirudins bestimmt. Es zeigt sich , daß die Sequenz vollständig intakt ist und mit der Aminosäure Leucin beginnt. Dieses Ergebnis wurde durch das Programm zur Identifikation der putativen Signalpeptidaseerkennungsstelle vorausgesagt. Überraschend ist aber die schlechte Ausbeute.

### Beispiel 7: Synthese des Fusionsgenes aus Leu-Hirudin und der Signalsequenz des Vorläufers der alkalischen Phosphatase aus E. fergusonii

Die Konstruktion erfolgt entsprechend dem in Beispiel 1 beschriebenen Schema mit Ausnahme, daß anstelle der Primer smompaf1 / f2 zwei neue Primer benutzt werden , die bezüglich ihrer Spezifität für das Hirudingen und ihrer Sequenz zur Erkennung durch das Restriktionsenzym *Eco*RI die gleichen Merkmale wie die smompa - Primer aufweisen, aber für die gewünschte Signalsequenz des alkalischen Phosphataseproteins aus *E. fergusonii* (Du Bose, R.F. and Hartl, D.L. Mol. Biol. Evol. 7, 547-577 (1990)) kodieren.

Diese Signalsequenz unterscheidet sich an fünf Positionen von der der alkalischen Phosphatase aus *E*. *coli.*

Primer fergusf1 hat folgende Sequenz :
5' GCTGAGCTGC CTGATCACCC CGGTGTCCCA GGCGcttacg tatactgact gca 3' [SEQ ID NO.: 14]

Primer fergusf2 hat die Sequenz :
5' ttttttgaat tcATGAAACA GAGCGCGATC GCGCTGGCTC TGCTgAGCTG CCTGATC 3' [SEQ ID NO.: 15]

Die beiden Primer sind bzgl. der Codonwahl für *E.coli* optimiert, d.h. sie entsprechen nicht vollständig der natürlichen Sequenz des Ausgangsgenes. Dabei wird der Primer fergusf1 entsprechend Beispiel 1 in PCR1 und der Primer fergusf2 entsprechend in der PCR2 verwendet. Die Expression wird im Vergleich zur Ala - Hirudinexpression mit Stamm *E. coli* WCM100 / pCM7053 durchgeführt. Es zeigt sich , daß die erzielte Expressionausbeute nur ein Bruchteil der im Vergleichsversuch erzielten Ausbeute beträgt. Sie ist nochmals um etwa die Hälfte geringer, als dies für die mit dem Konstrukt aus Signalpeptid von *E.coli* alkalischer Phosphatase und Leu-Hirudin beobachtet wird.

### Beispiel 8 : Synthese des Fusionsgenes aus Leu-Hirudin und der Signalsequenz des Vorläufers der Cyclodextrin Glucanotransferase aus Paenibacillus macerans

Die Konstruktion erfolgt entsprechend dem in Beispiel 1 beschriebenen Schema mit Ausnahme, daß anstelle der Primer smompaf1 / f2 zwei neue Primer benutzt werden , die bezüglich ihrer Spezifität für das Hirudingen und ihrer Sequenz zur Erkennung durch das Restriktionsenzym *Eco*RI die gleichen Merkmale wie die smompa -Primer aufweisen, aber für die gewünschte Signalsequenz des Cyclodextrin Glucanotransferase Genes aus *Paenibacillus macerans* (Takano,T., Fukuda,M., Monma,M., Kobayashi,S., Kainuma,K. and Yamane,K. J. Bacteriol. 166, 1118-1122 (1986)) kodieren.

Primer baccdgf1 hat folgende Sequenz :
5' CTTTCGCTGA GTATGGCGTT GGGGATTTCA CTGCCCGCAT GGGCActtac gtatactgac tgca 3' [SEQ ID NO.: 16]

Primer baccdgf2 hat die Sequenz :
5' ttttttgaat tcATGAAATC GCGGTACAAA CGTTTGACCT CCCTGGCGCT TTCGCTGAGT ATGGC 3' [SEQ ID NO.: 17]

Dabei wird der Primer baccdgf1 entsprechend Beispiel 1 in PCR1 und der Primer baccdgf2 entsprechend in der PCR2 verwendet. Die Expression wird im Vergleich zur Ala - Hirudinexpression mit Stamm *E. coli* WCM100 / pCM7053 durchgeführt. Es zeigt sich , daß die erzielte Expressionausbeute ca. ¼ der im Vergleichsversuch erzielten Ausbeute beträgt. Das synthetisierte Hirudin verhält sich im Thrombinhemmtest wie Leu-Hirudin. Dies bedeutet, daß das Signalpeptid korrekt prozessiert wurde. Dies entspricht nicht der Erwartung aus der theoretischen Analyse, die auf einen um 8 Aminosäuren verlängerten oder alternativ um zwei Aminosäuren verkürzten N - Terminus hindeutete.

### Beispiel 9 : Synthese des Fusionsgenes aus Leu-Hirudin und der Signalsequenz des E. coli PCFO20 Fimbrillin Vorläuferproteins (fotA)

Die Konstruktion erfolgt entsprechend dem in Beispiel 1 beschriebenen Schema mit Ausnahme, daß anstelle der Primer smompaf1 / f2 zwei neue Primer benutzt werden , die bezüglich ihrer Spezifität für das Hirudingen und ihrer Sequenz zur Erkennung durch das Restriktionsenzym *Eco*RI die gleichen Merkmale wie die smompa -Primer aufweisen, aber für die gewünschte Signalsequenz des *E. coli* PCFO20 Fimbrillin Vorläuferproteins (Viboud, G.I., Jonson, G., Dean-Nystrom, E. and Svennerholm, A.M. Infect. Immun. 64 (4), 1233-1239 (1996)) kodieren.

Primer pcf1-ala hat folgende Sequenz :
5' TGGTTTCAGC TTTAGTAAGC GGGGTTGCAT TTGCTCTTAC GTATACTGAC TGCAC 3' [SEQ ID NO.: 18]

Primer p-pcf2 hat die Sequenz :
5' TTTTGGGAAT TCATGAAAAA GACAATTATG TCTCTGGCTG TGGTTTCAGC TTTAGTAAGC 3' [SEQ ID NO.: 19]

Dabei wird der Primer pcf1-ala entsprechend Beispiel 1 in PCR1 und der Primer p-pcf2 entsprechend in der PCR2 verwendet. Die Expression wird im Vergleich zur Ala - Hirudinexpression mit Stamm *E. coli* WCM100 / pCM7053 durchgeführt. Es zeigt sich , daß die erzielte Expressionausbeute ca 40% der im Vergleichsversuch erzielten Ausbeute beträgt.

### Beispiel 10 : Synthese des Fusionsgenes aus Leu-Hirudin und der Signalsequenz des S. typhimurium Outer Membrane Protein (fimD)

Die Konstruktion erfolgt entsprechend dem in Beispiel 1 beschriebenen Schema mit Ausnahme, daß anstelle der Primer smompaf1 / f2 zwei neue Primer benutzt werden , die bezüglich ihrer Spezifität für das Hirudingen und ihrer Sequenz zur Erkennung durch das Restriktionsenzym *Eco*RI die gleichen Merkmale wie die smompa -Primer aufweisen, aber für die gewünschte Signalsequenz des S. *typhimurium* Outer Membrane Proteins (Rioux, C.R., Friedrich, M.J. and Kadner, R.J.; J. Bacteriol. 172 (11), 6217-6222 (1990)) kodieren.

Primer styfimf1 hat folgende Sequenz :
5' CGGCGCTGAG TCTCGCCTTA TTTTCTCACC TATCTTTTGC Ccttacgtat actgactgca 3' [SEQ ID NO.: 20]

Primer styfimf2 hat die Sequenz :
5' ttttttgaat tcaTGTCATT TCATCACCGG GTATTTAAAC TGTCGGCGCT GAGTCTC 3' [SEQ ID NO.: 21]

Dabei wird der Primer styfimf1 entsprechend Beispiel 1 in PCR1 und der Primer styfimf2 entsprechend in der PCR2 verwendet. Die Expression wird im Vergleich zur Ala - Hirudinexpression mit Stamm *E*. *coli WCM100* / pCM7053 durchgeführt. Es zeigt sich , daß die erzielte Expressionausbeute ca 10% der im Vergleichsversuch erzielten Ausbeute beträgt.

### Beispiel 11: Expression in E. coli

Das Beispiel beschreibt die Expression des Hirudin. Dazu werden je 1 -5ml LB-Medium, das 25mg/l Ampicillin enthält und 0,5 - 2mM IPTG (Isopropyl β-D-Thiogalactopyranoside) mit Zellen einer Transformante beimpft und ca. 20 Stunden bei 28°C im Brutschüttler bei 220 rpm geschüttelt. Anschließend wird nach Bestimmung der optischen Dichte die Zellsuspension zentrifugiert und Hirudin aus dem klaren Überstand bestimmt.

Parallel zur Expression von Refludan wird die Expression des in dem europäischen Patent 0 448 093 beschriebene Ala- Hirudin über das Plasmid pCM7053 in der in dem Patent beschriebenen Sekretormutante WCM100 durchgeführt. Damit wird ein direkter Vergleich der Expressionsrate möglich.

Die Expression im größeren Maßstab kann entsprechend dem US Patent 5,616,476 erfolgen. Refludan kann dann entsprechend der in diesem Patent in den Beispielen 5 und 6 beschrieben Methoden gereinigt werden.

### Beispiel 12 : Bestimmung der Hirudinkonzentration

Die Bestimmung der Hirudinkonzentration wird entsprechend der Methode von Grießbach *et al.* (Thrombosis Research 37, 347 -350, 1985) durchgeführt. Dazu werden definierte Mengen eines Refludanstandards zur Erstellung einer Eichkurve in die Meßreihe mit einbezogen. Damit kann die Ausbeute direkt in mg /I angegeben werden.

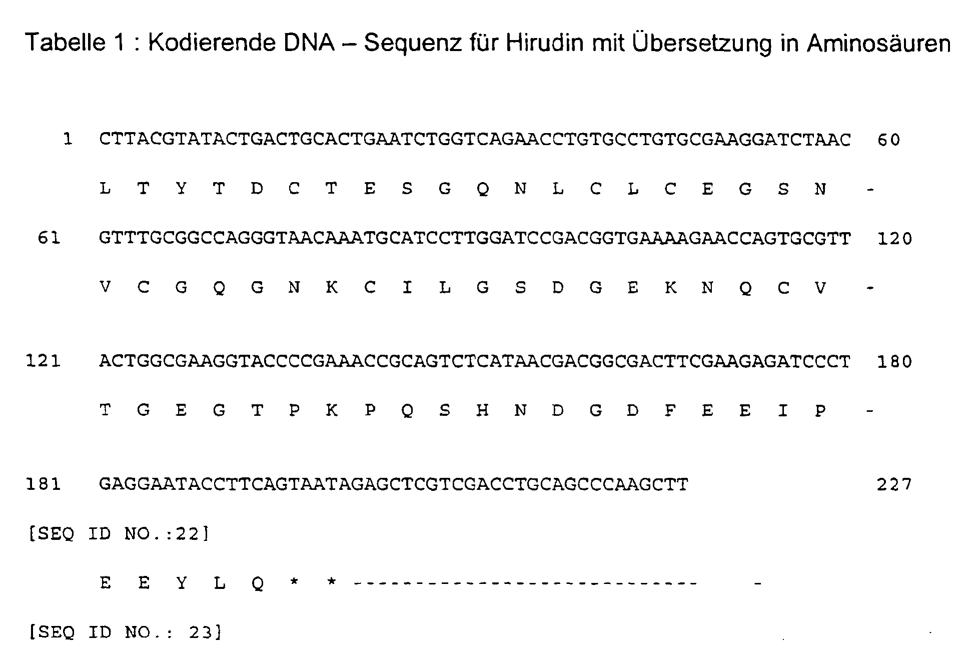

**Tabelle 2:**

| Beispiel | Signalsequenz | Primärstruktur | Relative Ausbeute pro ml Kultur | SEQ ID NO.: |
|---|---|---|---|---|
| - | Kontrolle : cgtase -Ala - Hirudin | MKRNRFFNTS AAIAISIALNTFF CSMQTIA | 1 | 24 |
| 1 | äußeres Membranprotein / *Serrtia marcescens* | MKKTAIALAVALAGFATVAQ A | 1,5 | 25 |
| 2 | oprF - Protein / *Pseudomonas fluorescens* | MKNTLGLAIGSLIAATSFGV LA | 1,1 | 26 |
| 3 | lambB -Protein / *E.coli* | MMITLRKLPL AVAVAAGVMS AQAMA | 1 | 27 |
| 4 | Fumat Reduktase / *Shewanella putrifaciens* | MKKMNLAVCI ATLMGTAGLM GTAVA | 1,5 | 28 |
| 5 | β-Lactamase / pBR322 | MSIQHFRVAL IPFFAAFSLPVFA | 0.5 | 29 |
| 8 | alk. Phosphatase / *E.coli* | MKQSTIALAL LPLLFTPVTK A | 0,1 | 30 |
| 9 | alk. Phosphatase / *E*. *fergusonii* | MKQSAIALAL LSCLITPVSQ A | 0,05 | 31 |
| 10 | Cyclodextrin Glucanotransferase / *Paenibacillus macerans* | MKSRYKRLTS LALSLSMALGI SLPAWA | 0,25 | 32 |
| 11 | Outer Membrane Protein / S. *typhimurium* | MSFHHRVFKL SALSLALFSH LSFA | 0,11 | 33 |

## Patentansprüche

1. Hirudinvorläufer enthaltend eine Signalsequenz ausgewählt aus der Gruppe enthaltend die Signalsequenzen des äußeren Membranproteins von *Serratia marcescens,* des oprF-Proteins von *Pseudomonas fluorescens* und der Fumarat-Reduktase von *Shewanella putrifaciens,* an welche C-terminal die Sequenz von Leu-Hirudin angefügt ist.

2. Hirudinvorläufer gemäß Anspruch 1**,** wobei die Signalsequenz ausgewählt wird aus der Gruppe enthaltend die Signalsequenz des äußeren Membranproteins von *Serratia marcescens* und der Fumarat-Reduktase von *Shewanella putrifaciens.*

3. Verfahren zur Herstellung von Leu-Hirudin, bei welchem als Zwischenstufe ein Hirudinvorläufer gemäß einem der Ansprüche 1 oder 2 vorkommt, bei dem
(a) ein Expressionsplasmid enthaltend eine DNA-Sequenz kodierend für den Hirudinvorläufer hergestellt wird;
(b) das Expressionsplasmid gemäß (a) in einer geeigneten *E. coli* Zelle exprimiert wird;
(c) der Hirudinvorläufer aus der *E*. *coli* sekretiert und gleichzeitig prozessiert wird; und
(d) das Leu-Hirudin aus dem Kulturmedium isoliert wird.

4. Verwendung eines Hirudinvorläufers gemäß einem der Ansprüche 1 oder 2 zur Herstellung von Leu-Hirudin.

5. Verwendung eines Hirudinvorläufers gemäß Anspruch 4 in einem Verfahren gemäß Anspruch 3.

## Claims

1. A hirudin precursor comprising a signal sequence selected from the group comprising the signal sequences of the outer membrane protein of *Serratia marcescens,* the oprF protein of *Pseudomonas fluorescens,* and the fumarate reductase of *Shewanella putrifaciens,* onto which there is C-terminal attachment of the sequence of Leu-hirudin.

2. A hirudin precursor as claimed in claim 1, where the signal sequence is selected from the group comprising the signal sequence of the outer membrane protein of *Serratia marcescens* and the fumarate reductase of *Shewanella putrifaciens.*

3. A process for preparing Leu-hirudin, in which a hirudin precursor as claimed in either of claims 1 or 2 occurs as intermediate, in which
(a) an expression plasmid comprising a DNA sequence coding for the hirudin precursor is prepared;
(b) the expression plasmid from (a) is expressed in a suitable *E. coli* cell;
(c) the hirudin precursor is secreted from the *E. coli* and simultaneously processed; and
(d) the Leu-hirudin is isolated from the culture medium.

4. The use of a hirudin precursor as claimed in either of claims 1 or 2 for preparing Leu-hirudin.

5. The use of a hirudin precursor as claimed in claim 4 in a process as claimed in claim 3.

## Revendications

1. Précurseur d'hirudine contenant une séquence signal choisie parmi le groupe contenant les séquences signal de la protéine membranaire extérieure de *Serratia marcescens,* de la protéine oprF de Pseudomonas *fluorescens* et de la fumarate-réductase de *Shewanella putrifaciens,* la séquence de Leu-hirudine étant jointe à l'extrémité C-terminale.

2. Précurseur d'hirudine suivant la revendication 1, dans lequel la séquence signal est choisie parmi le groupe contenant la séquence signal de la protéine membranaire extérieure de *Serratia marcescens* et de la fumarate-réductase de *Shewanella putrifaciens.*

3. Procédé de préparation de Leu-hirudine, dans lequel apparaît, comme étape intermédiaire, un précurseur d'hirudine suivant l'une des revendications 1 ou 2, procédé dans lequel
(a)un plasmide d'expression contenant une séquence d'ADN codant pour le précurseur d'hirudine est préparé,
(b)le plasmide d'expression selon (a) est exprimé dans une cellule appropriée d'*E*. *coli,*
(c)le précurseur d'hirudine est sécrété à partir de *E. coli* et traité simultanément, et
(d)la Leu-hirudine est isolée à partir du milieu de culture.

4. Utilisation d'un précurseur d'hirudine suivant l'une des revendications 1 ou 2, pour la préparation de Leu-hirudine.

5. Utilisation d'un précurseur d'hirudine suivant la revendication 4, dans un procédé suivant la revendication 3.
